# Europäisches Patentamt

## European Patent Office

## Office européen des brevets

(11) Publication number: **0 145 775**
**B1**

(12) **EUROPEAN PATENT SPECIFICATION**

(45) Date of publication of patent specification: **08.02.89**

(21) Application number: **84902545.7**

(22) Date of filing: **13.06.84**

(86) International application number:
**PCT/SE84/00222**

(87) International publication number:
**WO 84/04887 20.12.84 Gazette 84/30**

(51) Int. Cl.⁴: **A 61 K 45/02, A 61 K 35/16 //
A61M1/00**

(54) **A METHOD OF TREATING INTERFERON SENSITIVE DISEASES, AND A METHOD AND A DEVICE FOR PREPARING A -g(g)-INTERFERON CONTAINING PREPARATION.**

(30) Priority: **13.06.83 US 503575**

(43) Date of publication of application:
**26.06.85 Bulletin 85/26**

(45) Publication of the grant of the patent:
**08.02.89 Bulletin 89/06**

(84) Designated Contracting States:
**AT BE CH DE FR GB LI LU NL SE**

(56) References cited:
**WO-A-83/01899**
**US-A-4 276 282**
**Läkartidningen, vol. 77, pp. 4380-82, publ. 1980
(Hyden H. et al.) "Interferonbehandling av
tumörer med extrakorporal perfusion"**

**Chemical Abstracts, vol. 98, abstract 87397e,
publ. 1983, Vopr. Virusol. 1982 (5), 531-4**
**Chemical Abstracts, vol. 96, abstract 96 936r,
publ. 1982, Methods Enzymol. 1981, 78
(Interferous, PtA), 236-42**

(73) Proprietor: **Lindblom, Ragnvald Erik
Alsäter
S-740 10 Almunge (SE)**
(73) Proprietor: **Rothman, Ulf Sven Erik
Östergatan 49
S-230 10 Skanör (SE)**

(72) Inventor: **Lindblom, Ragnvald Erik
Alsäter
S-740 10 Almunge (SE)**
Inventor: **Rothman, Ulf Sven Erik
Östergatan 49
S-230 10 Skanör (SE)**

(74) Representative: **Kummelsten, Per-Arne et al
UPPSALA PATENTBYRA P.O. Box 9013
S-750 09 Uppsala (SE)**

Courier Press, Leamington Spa, England.

## Description

The present invention relates to a novel method of producing interferon and novel means for carrying out this method.

Interferons are proteinaceous substances which are induced intra cellularly or extra cellularly upon exposure of the cells to interferon inducing agents such as viruses, bacteria, protozoas, rickettsia, nucleic acids, endotoxines, polysaccharides;

Interferons have a great potential interest as drugs since they i.a. unspecifically inhibit the growth of various viruses in the cells, have an antitumour effect on non-viral or viral tumours;

However, the development of interferon as a drug is severely inhibited by the great difficulties in preparing the necessary amounts, i.a. depending on the fact that interferon is species specific. Thus, only interferon originating from live human cells is useful for human therapy. For the preparation of interferon human leukocytes and the like are conventionally used, and the very limited supply of such starting material is a great problem in view of the small amounts which can be prepared.

Attempts have also been made to prepare interferon *in vitro* by culturing established human cells on a nutrient medium in the presence of various interferon inducers. However, this method has not given the desired results.

In recent years it has been established that human interferon exists in at least three different molecular variants, viz. α-interferon (previously called leukocyte interferon), β-interferon (previously fibroblast interferon), and γ-interferon (previously immune interferon).

It is further known from numerous publications that many substances induce the formation of interferons. Thus, α- and β-interferon are induced by viruses, and γ-interferon by so called mitogens and antigens.

γ-interferon is believed to have a tremendous potential as an agent for treating interferon-sensitive diseaess, such as tumours. However, γ-interferon is difficult to produce and is unstable, and attempts to stabilize it have so far been unsuccessful.

WO—A—8.301.899 discloses a process for the production of human γ-interferon by separating the leukocyte fraction of blood, removing the erythrocytes, suspending the leukocytes in a nutrient medium, treating the leukocytes with α- or β-interferon, then treating the same with a mitogenic agent, and separating the interferon-containing liquid from the cells.

US—A—4,276,282 discloses another process for the production of human-specific interferon comprising cultivation of established human cells such as leukocytes and exposure of the same to an interferon inducer, wherein the established human cells are cultivated in other warm-blooded animal bodies by either transplanting the cells thereinto or inoculating a diffusion chamber containing the cells into an animal, while allowing the animal to supply the cells with its nutrient body fluid.

The present invention suggests a new approach to the problem of preparing and using γ-interferon for treating interferon-sensitive diseases. A first aspect of the invention is based on the *per se* well known fact that mitogens induce the production of γ-interferon, and in accordance with this aspect of the invention γ-interferon production is induced by incubating whole blood samples from a patient to be treated with one or more mitogens *in vitro*. After the incubation has been terminated the incubated blood sample is subjected to a separation step to produce a plasma containing the interferon produced but not the mitogen. The incubation and separation steps are performed *in vitro*.

The (γ) interferon-containing plasma is then re-administered to the same patient, who will thus receive an interferon preparation which does not only originate from the same species (a human) but from the same individual (himself/herself): the preparation is "individual specific". This procedure offers a plurality of important advantages, i.a. the following: a very simple, rapid and inexpensive production of the preparation, elimination or considerable reduction of the instability problems with γ-interferon, reduced side effects because of the individual specificity.

It is essential to separate the mitogen before re-administering the interferon containing plasma to the patient. For this separation any suitable conventional separation technique can be used, provided that the same is capable of separating all of the mitogen from the plasma product while leaving a therapeutically effective amount of the produced interferon therein. The separation technique can be based on the physical and/or biological/biochemical properties of the mitogen. Examples of suitable separation techniques are affinity chromatography, e.g. using biotine, and ultrafiltration, the latter technique being described in some detail below as an illustrative example only.

When using ultrafiltration the cut-off properties of the ultra filter are chosen with regard to the choice of the γ-interferon inducing mitogens. Thus, the filter and the mitogens should be chosen such that γ-interferon can pass through the filter, whereas the mitogens are excluded, or vice versa. Since the major part of γ-interferon has a molecular weight of about 20,000—75,000 (with a minor part having a molecular weight of about 65,000—70,000) the filter should permit substances of a molecular weight of up to about at least 50,000 to pass through, when the mitogen has a molecular weight which is higher than that of the interferon. The upper cut-off limit is chosen with regard to the molecular weight of the mitogen used. Obviously the mitogen must in this case have a molecular weight of at least about 50,000. Preferred mitogens are phytohemagglutinin (PHA) and concanvaline-A (con-A). PHA has a molecular weight of about 127,000, thus permitting a cut-off value of the filter of e.g. about 100,000, which also permits the minor part

(see above) of γ-interferon to pass through the filter. Mitogens having comparatively low molecular weights, e.g. overlapping the molecular weight range for the interferon, can be bound to a suitable matrix to increase the molecular weight and permit separation by ultrafiltration. As mentioned above low molecular mitogens can be separated by using a filter having a low cut-off limit permitting the mitogen, but not the interferon to pass through.

Mitogens having lower molecular weights can also be used when separating the mitogen by other techniques than filtration. A preferred mitogen of this molecular weight size is Staph. enterotoxin.

The incubation of the whole blood sample with the mitogen (or mitogens) is carried out under conditions promoting γ-interferon production, e.g. at a temperature of about 35 to 40°C and for e.g. at least about 2 or 4 hours (up to several days). The amount of mitogen to be used depends on the specific mitogen. This amount is chosen so as to produce an optimal amount of γ-interferon, and it can easily be established by a person of average skill in the art by simple tests. Excessive amounts of mitogen should be avoided since this may inhibit the interferon production. For the preferred mitogen PHA suitable amounts are e.g. from about 1 to about 5 μg PHA per ml whole blood sample.

The whole blood sample is preferably taken by means of a specially prepared sterile test tube which can be provided with a needle for taking a blood sample directly from the patient to the tube. Such a test tube—which is preferably heparinized or provided with any other suitable anti-coagulant and contains an efficient amount of one or more mitogens—is also an object of the present invention. The incubated blood sample is preferably centrifugated before the separation step, especially the filtering step, in order to prevent clotting of the filter by erythrocytes etc. The filter may be provided in the test tube, or the separation may be performed using separate separation means such as an ultrafiltration apparatus.

The preferred mode of administering the interferon-containing preparation is by intramuscular injection, but also other routes may be possible, such as intravenous or subcutaneous injection. Where combined treatment with a histamine H2-antagonist is used, the histamine H2-antagonist may be injected together with the γ-interferon-containing plasma or it may be administered separately, e.g. by the oral route. The dosage of the histamine H2-antagonist will vary depending on the chosen compound, the condition of the patient, etc. As a thumb rule one can use dosages of the same order as those used when treating e.g. peptic ulcer with the histamine H2-antagonist alone, these dosages being well known.

It is believed that the effect obtained by means of the interferon preparation and treatment in accordance with the invention is a result both of the direct effect of γ-interferon as such and on the fact that γ-interferon triggers the production of α- and β-interferon in vitro and/or in vivo. It may in this context be mentioned that α- and β-interferon are capable of passing through the filter together with γ-interferon (when using this separation technique). The incubation of the whole blood sample with the mitogen also triggers the production of other lymphokines, which further enhance the effect of the γ-interferon-containing blood plasma. In particular the triggered production of interleukins, especially interleukin II (ILII), is believed to give a valuable contribution in the treatment of the above mentioned diseases. Such lymphokins, especially ILII, can readily be separated from the mitogen in the separation step, together with the interferons. For example ILII has a suitable molecular weight somewhat lower than that of γ-interferon.

The interferon-containing plasma prepared by the claimed method may be administered in combination with a histamine H2-antagonist. As is well known, histamine H2-antagonists are compounds which block histamine H2-receptors. Histamine H2-antagonists are useful i.a. for the treatment of peptic ulcer. The most well known histamine H2-antagonist is cimetidine (N - cyano - N' - methyl - N'' - [2 - [[(5 - methyl - 1H - imidazol - 4 - yl)methyl]thio]ethyl]guanidine), but a great number of other histamine H2-antagonists have been described. It has unexpectedly been found that a synergistic effect, in particular a synergistic anti-tumour effect, is achieved when γ-interferon is administered together with a histamine H2-antagonist such as cimetidine. The mechanism of this synergistic effect has not been clarified.

The following test procedures were carried out in order to verify the operativeness of the method according to the invention. The Examples are given for illustrative purposes only, and they are not intended to limit the scope of the invention.

Example 1

5 ml of human whole blood were collected in a sterile heparinized (as an anti-coagulant) plastic vacuum tube containing 15 μg PHA (3 μg/ml blood sample) and incubated at 37°C on a water bath for at least 4 h. The tube was then centrifugated and the supernatant plasma collected (about 2.5 ml). The collected plasma was then, still under sterile conditions, ultrafiltrated through an Amicon Diaflo (registered trade mark) filter. Two types of this filter were used, viz. the XM 50 type and the XM 100A type, having cut-off limits of 50,000 and 100,000 respectively. This means that molecules having a molecular weight up to about 50,000 and 100,000 respectively can pass through the filter, whereas larger molecules are excluded.

The ultrafiltrate obtained was tested as to interferon activity in the so-called NK system (as described by Ratliff et al in Cellular Immunology, Vol. 57, Jan. 1, 1981) with the use of targert cell K 562 erythroid cell line. It was found that the ultrafiltrate exhibited NK activity even after so high dilution as 1:10,000. A 100% increase compared to the normal value was observed as the highest NK activity. No mitogen (PHA) could be detected in any of the tested ultrafiltrates, and no difference was found between the ultrafiltrates from the two filters (XM 50 and XM 100A).

Example 2

PHA in varying amounts was added to 10 ml of heparinized peripheral blood, mixed well and incubated for 4 h at 37°C. The blood cells were separated from plasma by centrifugation, and the plasma was ultrafiltrated through Amicon filters with a cut-off of MW. 50,000. Filtered plasma was tested for capacity to simulate the NK (natural killer) activity of mononuclear blood or spleen cells as an indicator of the presence of gamma-interferon. The test was a standard test for NK lymphocyte activity making use of target cells from a very sensitive cell line (K 562 erythroblastoid cells), labelled with $^{51}$Cr. Mononuclear cells of healthy human donor were incubated for 2 h at 37°C with plasma to be tested for interferon content, after which the cells were added to K 562 target cells at various ratios and incubated for 4 h. The released radioactivity was measured in a gamma counter as a measure of the extent of cytolytic effects on the target cells.

Results of this test are given in Table 1, from which it can be seen that plasma of heparinized human blood which has been incubated for 4 h with 2 or 8 μg PHA per ml leads to approximately a doubling of the NK activity when tested at dilution 1:30 and 1:100 and compared to plasma of similarly incubated blood without added PHA. Similar tests, but using rat blood instead of human blood samples demonstrated a stimulated NK activity of spleen lymphocytes exposed to plasma diluted 1:30, 1:100, 1:1000 and 1:10,000.

Example 3

γ-interferon-containing rat plasma was prepared as in Example 2, using PHA (available from Pharmacia AB, Uppsala, Sweden) as the mitogen. The γ-interferon preparation was injected intramuscularly (21 doses of 0.1—0.4 ml per animal) in combination with a histamine H2-antagonist (cimetidine) to rats which had been challenged subcutaneously with a transplantable DMH-induced colon carcinoma isograft ($1.5 \times 10^3$ viable cells). The rejection of colon cancer isograft was evaluated by the number of tumor-free rats after 3, 6 and 13 weeks respectively. The treated rats demonstrated a significant reduction in tumour outgrowth compared both to untreated controls and to animals treated with the histamine H2-antagonist alone.

TABLE 1

Stimulation of NK activity of human mononuclear blood cells by plasma of blood samples incubated with various doses of PHA for 4 h at 37°C.

| Preincubation of effector cells | | | Specific $^{51}$Cr release at ratio | | | Lytic units[1] per $10^7$ |
|---|---|---|---|---|---|---|
| Type of plasma | | Dilution | 20:1 | 10:1 | 5:1 | |
| Medium | | — | 33.2 | 10.8 | 8.2 | 9.9 |
| Plasma control | | 1:100 | 38.1 | 28.6 | 20.1 | 18.4 |
| Plasma of blood | +32 μg PHA-LS[2] | 1:100 | 38.8 | 35.3 | 15.1 | 19.7 |
| „ | +8 μg „ | 1:100 | 47.6* | 41.9* | 30.3* | 44.3 |
| „ | +2 μg „ | 1:100 | 48.9* | 32.1 | 23.3 | 25.9 |
| „ | +0.5 μg „ | 1:100 | 39.4 | 26.4 | 19.2 | 17.8 |
| „ | +0.13 μg „ | 1:100 | 36.8 | 24.4 | 15.6 | 15.2 |
| Medium | | — | 33.2 | 10.8 | 8.2 | 9.9 |
| Plasma control | | 1:30 | -43.4 | 33.7 | 22.9 | 25.1 |
| Plasma of blood | +32 μg PHA-LS | 1:30 | 46.4 | 32.9 | 15.5 | 21.5 |
| „ | +8 μg „ | 1:30 | 25.1 | 24.0 | 12.9 | 6.9 |
| „ | +2 μg „ | 1:30 | 67.5** | 49.1** | 29.1 | 39.0 |
| „ | +0.5 μg „ | 1:30 | 33.4 | 28.2 | 25.7 | 17.0 |
| „ | +0.13 μg „ | 1:30 | 40.5 | 31.8 | 23.4 | 23.3 |

[1] One lytic unit is the number of effector cells required to result in 30% specific $^{51}$Cr-release.
[2] Blood was incubated with the indicated amount of PHA per ml for 4 h at 37°C. Plasma separated by centrifugation was ultrafiltered to remove PHA.
*=p<0.05 in Student's t-test    **=p<0.01 in Student's t-test

## EP 0 145 775 B1

**Claims**

1. A method of preparing a γ-interferon-containing preparation, comprising the steps of incubating *in vitro* a whole blood sample, taken from a patient to be treated with said γ-interferon preparation, in the presence of a mitogen to produce γ-interferon in the whole blood sample, and subjecting said whole blood sample to a separation step so as to produce a blood plasma preparation which is free of said mitogen, contains a therapeutically effective amount of γ-interferon, and is suitable for re-administration to the patient from which said whole blood sample is taken.

2. The method of Claim 1, wherein said separation step comprises centrifugation of said incubated whole blood sample for separating the blood plasma, and ultrafiltration of said blood plasma for removing said mitogen therefrom.

3. The method of Claim 1, wherein said separation step comprises separation of said mitogen by affinity chromatography.

4. The method of any one of Claims 1—3, wherein said mitogen is bound to a suitable matrix.

5. The method of any one of Claims 1—4, wherein said mitogen is selected from phytohemagglutinin (PHA), concanvaline A (Con-A), and Staph. enterotoxin.

6. The method of any one of the preceding claims, wherein said blood plasma preparation is suitable for administration to the patient by injection.

7. The method of any one of Claims 1 to 6, wherein said incubation of said whole blood sample also produces at least one lymphokin other than interferon, especially interleukin II, and wherein said separation step is carried out so as to retain at least a major part of said at least one lymphokin, especially interleukin II, in said plasma preparation.

8. A sterile sample container suitable for *in vitro* preparation of γ-interferon, said container being provided with means for taking a whole blood sample from a human patient and filling said container therewith under sterile conditions, said container containing a mitogen in an amount sufficient for producing γ-interferon upon incubation together with said whole blood sample, and said container optionally comprising filter means for separating said mitogen, so as to produce a blood plasma preparation which is free of said mitogen, contains a therapeutically effective amount of γ-interferon, and is suitable for re-administration to the patient from which said blood sample is taken.

9. The container of Claim 8, which further contains a therapeutically effective amount of a histamine H2-antagonist.

10. The container of Claim 9, wherein said histamine H2-antagonist is a guanidine type histamine H2-antagonist.


**Patentansprüche**

1. Verfahren zur Herstellung eines γ-Interferon enthaltenden Präparats, dadurch gekennzeichnet, daß man eine Gesamtblutprobe eines Patienten, der mit dem γ-Interferonpräparat behandelt werden soll, in Gegenwart eines Mitogens zur Bildung von γ-Interferon in der Gesamtblutprobe *in vitro* inkubiert und die Gesamtblutprobe in der Weise einer Trennungsstufe unterzieht, daß ein von dem Mitogen freies Blutplasmapräparat gebildet wird, welches eine therapeutisch wirksame Menge an γ-Interferon enthält und für die Wiederverabreichung an den Patienten, von dem die Gesamtblutprobe stammt, geeignet ist.

2. Verfahren nach Anspruch 1, dadurch gekennzeichnet, daß man bei der Trennungsstufe die inkubierte Gesamtblutprobe zur Abtrennung des Blutplasmas zentrifugiert und das Blutplasma zur Entfernung des Mitogens einer Ultrafiltration unterzieht.

3. Verfahren nach Anspruch 1, dadurch gekennzeichnet, daß man bei der Trennungsstufe das Mitogen auf dem Wege der Affinitätschromatographie abtrennt.

4. Verfahren nach einem der Ansprüche 1 bis 3, dadurch gekennzeichnet, daß man das Mitogen an eine geeignete Matrix bindet.

5. Verfahren nach einem der Ansprüche 1 bis 4, dadurch gekennzeichnet, daß das Mitogen aus der Phytohämagglutinin (PHA), Concanvalin A (Con-A) und Staph. enterotoxin umfassenden Gruppe gewählt wird.

6. Verfahren nach einem der vorangegangenen Ansprüche, dadurch gekennzeichnet, daß das Blutplasmapräparat für die Verabreichung an den Patienten durch Injektion geeignet ist.

7. Verfahren nach einem der Ansprüche 1 bis 6, dadurch gekennzeichnet, daß bei der Inkubation der Gesamtblutprobe ebenfalls mindestens ein von Interferon verschiedenes Lymphokin, insbesondere Interleukin II, gebildet wird, wobei die Trennungsstufe in der Weise durchgeführt wird, daß mindestens der Hauptanteil des zumindest einen Lymphokins, insbesondere Interleukin II, in dem Plasmapräparat zurückbleibt.

8. Steriler Probenbehälter für die *in vitro*-Herstellung von γ-Interferon, wobei der Behälter mit einer Vorrichtung zur Entnahme mit und Aufnahme von der Gesamtblutprobe eines Patienten unter sterilen Bedingungen ausgestattet ist, welcher Behälter ein Mitogen in ausreichender Menge zur Bindung von γ-Interferon bei der Inkubation mit der Gesamtblutprobe enthält und gegebenenfalls eine Filtriervorrichtung zur Abtrennung des Mitogens und damit der Herstellung eines Mitogen-freien

5

# EP 0 145 775 B1

Blutplasmapräparats, das eine therapeutisch wirksame Menge an γ-Interferon enthält und für die Wiederverabreichung an den Patienten, von dem die Blutprobe stammt, geeignet ist, aufweist.

9. Behälter nach Anspruch 8, dadurch gekennzeichnet, daß er weiterhin eine therapeutisch wirksame Menge eines Histamin-H2-Antagonisten enthält.

10. Behälter nach Anspruch 9, dadurch gekennzeichnet, daß der Histamin-H2-Antagonist als Histamin-H2-Antagonist vom Guanidintyp vorliegt.

## Revendications

1. Procédé de production d'une préparation contenant du γ-interféron, comprenant les étapes d'incubation *in vitro* d'un échantillon de sang entier, prélevé chez un patient devant être traité avec cette préparation de γ-interféron, en présence d'un mitogène pour produire du γ-interféron dans l'échantillon de sang entier, et de soumission de cet échantillon de sang entier à une étape de séparation pour produire une préparation de plasma sanguin qui est dépourvue de ce mitogène, contient une quantité de γ-interféron efficace du point de vue thérapeutique, et convient pour être ré-administrée au malade chez qui a été prélevé cet échantillon de sang entier.

2. Procédé suivant la revendication 1, dans lequel l'étape de séparation comprend la centrifugation de cet échantillon de sang entier pour séparer le plasma sanguin, et l'ultrafiltration de ce plasma sanguin pour en éliminer ce mitogène.

3. Procédé suivant la revendication 1, dans lequel l'étape de séparation comprend la séparation de ce mitogène par chromatographie d'affinité.

4. Procédé suivant l'une quelconque des revendications 1 à 3, dans lequel ce mitogène est fixé à une matrice convenable.

5. Procédé suivant l'une quelconque des revendications 1 à 4, dans lequel ce mitogène est choisi parmi la phytohémagglutinine (PHA), la concanavaline (Con-A) et l'entérotoxine Staph.

6. Procédé suivant l'une quelconque des revendications 1 à 5, dans lequel cette préparation de plasma sanguin convient pour être administrée par injection au malade.

7. Procédé suivant l'une quelconque des revendications 1 à 6, dans lequel l'incubation de cet échantillon de sang entier produit aussi au moins une lymphokine autre que l'interféron, en particulier l'interleukine II et dans lequel cette étape de séparation est effectuée de façon à retenir au moins une partie majeure de cette lymphokine qui est au moins présente, en particulier l'interleukine II, dans cette préparation de plasma.

8. Récipient d'échantillon stérile convenable pour une préparation *in vitro* du γ-interféron, ce récipient étant pourvu de moyens pour prélever un échantillon de sang entier chez un malade et d'en remplir le récipient dans des conditions stériles, ce récipient contenant un mitogène en quantité suffisante pour produire le γ-interféron par incubation avec cet échantillon de sang entier, et ce récipient comprenant éventuellement des moyens de filtration pour séparer ce mitogène, de façon à produire une préparation de plasma sanguin qui est dépourvue de ce mitogène, contient une quantité efficace du point de vue thérapeutique du γ-interféron et convient pour être ré-administrée au malade chez qui l'échantillon de sang a été prélevé.

9. Récipient suivant la revendication 8, qui contient de plus une quantité efficace du point de vue thérapeutique d'un antagoniste d'histamine H2.

10. Récipient suivant la revendication 9, dans lequel cet antagoniste d'histamine H2 est un antagoniste d'histamine H2 de type guanidine.